# EUROPEAN PATENT SPECIFICATION

(11) **EP 4 086 339 B1**
(45) Date of publication and mention of the grant of the patent: **26.03.2025**
(21) Application number: 21744626.9
(22) Date of filing: 25.01.2021
(51) Int. Cl.: A61L 27/22, A61L 27/36, A61L 27/38, A61L 27/54, A61L 27/58, C12N 5/071, C12N 5/077, C12N 5/0775

(54) **BIOADHESIVE SHEET-LIKE MATERIAL FOR ADHESION TO ORGAN SURFACE**
BIOADHÄSIVES BLATTFÖRMIGES MATERIAL ZUR ADHÄSION AN EINER ORGANOBERFLÄCHE
MATÉRIAU STRATIFORME BIO-ADHÉSIF POUR ADHÉRENCE SUR UNE SURFACE D'ORGANE

(30) Priority: 24.01.2020 JP 2020010071
(43) Date of publication of application: 09.11.2022
(73) Proprietor: NAGASAKI UNIVERSITY, Nagasaki 852-8521 (JP); TERUMO Kabushiki Kaisha, Tokyo 151-0072 (JP)
(72) Inventor: MARUYA, Yasuhiro, Nagasaki-shi, Nagasaki 852-8521 (JP); YAMAGUCHI, Shun, Nagasaki-shi, Nagasaki 852-8521 (JP); KANETAKA, Kengo, Nagasaki-shi, Nagasaki 852-8521 (JP); HIGASHI, Miki, Nagasaki-shi, Nagasaki 852-8521 (JP); EGUCHI, Susumu, Nagasaki-shi, Nagasaki 852-8521 (JP); OHASHI, Fumiya, Ashigarakami-gun, Kanagawa 259-0151 (JP); MATSUMURA, Masaki, Ashigarakami-gun, Kanagawa 259-0151 (JP)
(74) Representative: Prüfer & Partner mbB Patentanwälte · Rechtsanwälte
(86) International application number: PCT/JP2021/002375
(87) International publication number: WO 2021/149830

(56) References cited:
- EP-A1- 3 895 754
- WO-A1-2006/093153
- WO-A1-2017/130802
- WO-A1-2020/235574
- JP-A- 2009 000 511
- JP-A- 2016 052 271
- US-A1- 2014 328 806
- Z. TANG ET AL: "Recent development of temperature-responsive surfaces and their application for cell sheet engineering", REGENERATIVE BIOMATERIALS, vol. 1, no. 1, 1 November 2014 (2014-11-01), pages 91 - 102, XP055362594, ISSN: 2056-3418, DOI: 10.1093/rb/rbu011
- NUMMI ANNU ET AL: "Epicardial delivery of autologous atrial appendage micrografts during coronary artery bypass surgery—safety and feasibility study", PILOT AND FEASIBILITY STUDIES, vol. 3, no. 1, 1 December 2017 (2017-12-01), XP055851412, Retrieved from the Internet <URL:https://pilotfeasibilitystudies.biomedcentral.com/track/pdf/10.1186/s40814-017-0217-9.pdf> DOI: 10.1186/s40814-017-0217-9

## Description

### Technical Field

The present invention relates to a bioadhesive sheet-shaped material, including an extracellular matrix layer, a sheet-shaped cell culture, and a biodegradable gel layer, in which the bioadhesive sheet-shaped material has the extracellular matrix layer on one surface and the biodegradable gel layer on the other surface with the sheet-shaped cell culture interposed therebetween, and is for use in a method of attaching the extracellular matrix layer onto a surface of an organ, wherein the organ is stomach, large intestine, duodenum, or liver, wherein the sheet-shaped cell culture contains myoblast cells or mesenchymal stem cells, and wherein the biodegradable gel layer is obtained by mixing a liquid containing fibrinogen with a liquid containing thrombin; as well as to the bioadhesive sheet-shaped material for use in a method for treating a disease that is ameliorated by application of the bioadhesive sheet-shaped material, comprising attaching a surface having an extracellular matrix layer of the sheet-shaped material in an effective amount of the bioadhesive sheet-shaped material to a specific organ of a subject in need thereof.

### Background Art

In recent years, in order to repair damaged tissues or the like, attempts to transplant various cells have been made. For example, for repairing myocardial tissues damaged due to an ischemic heart disease such as angina pectoris or myocardial infarction, attempts to utilize fetal cardiomyocytes, skeletal myoblast cells, mesenchymal stem cells, cardiac stem cells, embryonic stem (ES) cells, induced pluripotent stem (iPS) cells, and the like have been made (Non Patent Literature 1).

As an example of such attempts, sheet-shaped cell cultures, which are obtained by forming cells into a sheet shape, have been developed, and some of the sheet-shaped cell cultures are in the stage of clinical application. Examples of such clinical application include repair of impaired myocardium by means of implantation of engineered autologous myoblast sheets (Non Patent Literature 2), attachment of a cell sheet containing liver cells derived from mesenchymal stem cells onto a liver surface for the purpose of suppressing liver damage (Patent Literature 1), reduction of liver fibrosis by transplantation of IC-2-engineered mesenchymal stem cell sheet (Non Patent Literature 3), and a cell sheet composition for curing or preventing the leakage from a wound site of a hollow organ by attaching the cell sheet composition at the wound site of the hollow organ (Patent Literature 1).

Further, a sheet-shaped cell culture is fragile, and thus, it is difficult to use the sheet-shaped cell culture in operation in clinical application. For this reason, for example, a sheet-shaped cell culture having favorably improved adhesiveness to tissue (Patent Literature 3), a laminate of a fibrin gel and a sheet-shaped cell culture (Patent Literature 4), and the like have been proposed. Further, Non Patent Literature 4 highlights the recent development of temperature-responsive surfaces and their application for cell sheet engineering.
Non Patent Literature 5 describes micrograft cell sheets comprising atrial appendage-derived cells (AADCs).
Patent Literature 5 refers to a method for fabricating a three-dimensional artificial cardiac patch construct. Patent Literature 6 refers to a device for efficiently attaching a sheet-shaped object to a target site.
Patent Literature 7 refers to a laminate of a sheet-shaped cell culture as well as a method for preparing the same.

### Citation List

### Patent Literature

Patent Literature 1: JP 6008297 B2
Patent Literature 2: WO 2017/130802
Patent Literature 3: WO 2006/093153
Patent Literature 4: JP 6495603 B2
Patent Literature 5: US 2014/328806 A1
Patent Literature 6: EP 3895754 A1
Patent Literature 7: JP 2016052271 A

### Non Patent Literature

Non Patent Literature 1: Haraguchi et al., Stem Cells Trans1 Med. 2012 Feb; 1(2): 136-41
Non Patent Literature 2: Imran A. Memon et al., The Journal of Thoracic and Cardiovascular Surgery, Volume 130, Number 5, 1333-1341
Non Patent Literature 3: Noriko Itaba et al., SCIENTIFIC REPORT (2019) 9:6841
Non Patent Literature 4: Z. Tang et al., REGENERATIVE BIOMATERIALS (2014) 1: 91
Non Patent Literature 5: Nummi Annu et al., PILOT AND FEASIBILITY STUDIES (2017) vol. 3 no. 1

### Summary of Invention

### Technical Problem

Recognizing that development of a new sheet-shaped cell culture with higher operability is required for the further spread of regenerative medicine, an object of the present invention is to provide a bioadhesive sheet-shaped material having favorable bioadhesiveness onto a surface of an organ and is easy to operate in clinical application, a method for producing the bioadhesive sheet-shaped material, and a method for treating a disease by using the bioadhesive sheet-shaped material.

### Solution to Problem

In conducting intensive studies on the sheet-shaped cell culture that is engrafted well into various tissues, the present inventors have found for the first time that a myoblast sheet having an extracellular matrix layer on one surface and a biodegradable gel layer on the other surface can be engrafted well onto a surface of liver or large intestine by attaching a surface of the extracellular matrix layer to an organ, and as a result of further studies based on such a finding, the present inventors have completed the present invention.

That is, the present invention relates to the subject-matter as defined in the independent claims. Preferred embodiments are defined in the dependent claims.

### Advantageous Effects of Invention

By using the bioadhesive sheet-shaped material that is for use in a method of the present invention, favorable bioadhesiveness can be obtained when the bioadhesive sheet-shaped material containing a sheet-shaped cell culture is attached to an organ of a subject. Further, the bioadhesive sheet-shaped material for use in a method of the present invention can be more easily attached to an organ of a subject. In addition, in the attachment of the bioadhesive sheet-shaped material for use in a method of the present invention to an organ of a subject, a device for delivery can be used, and by a less invasive method for a subject, for example, a method of using a laparoscope, the bioadhesive sheet-shaped material can be attached to an organ of a subject. Moreover, the organ after the attachment of the bioadhesive sheet-shaped material can retain the favorable condition.

### Brief Description of Drawings

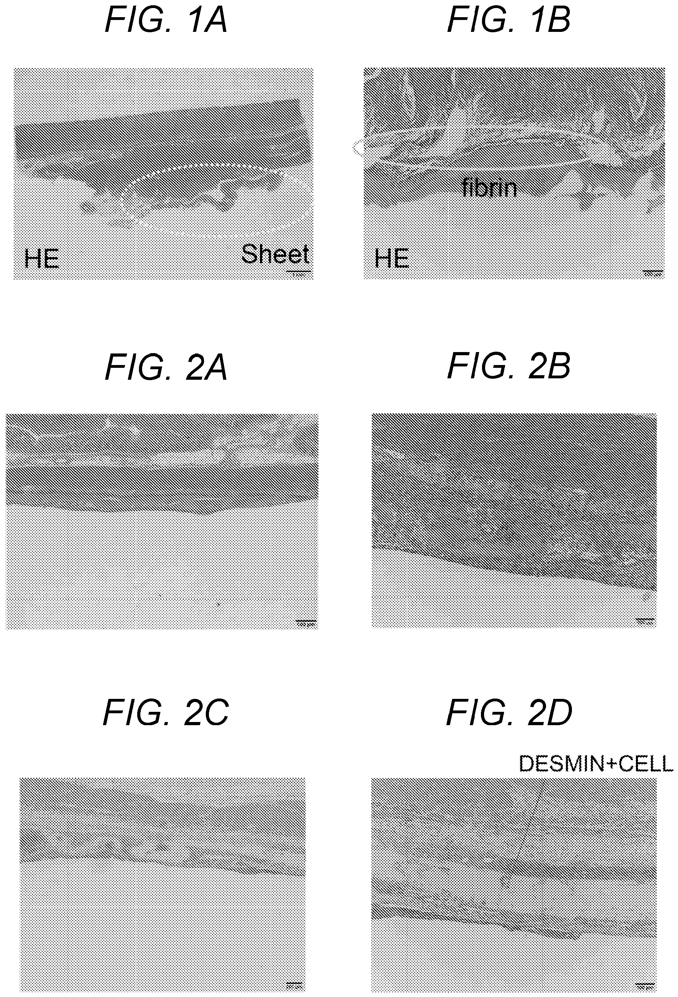

Figs. 1A and 1B each show a stained tissue image obtained by staining a tissue from the stomach to which a bioadhesive sheet-shaped material has been attached, with hematoxylin and eosin (HE). The part surrounded by a circle with a dashed line shows the attached bioadhesive sheet-shaped material. The part surrounded by a circle with a straight line shows a part indicating histological continuity. Fig. 1B shows a further enlarged part of Fig. 1A.
Figs. 2A and 2B each show a stained tissue image obtained by staining a tissue from the large intestine to which a bioadhesive sheet-shaped material has been attached, with hematoxylin and eosin. Fig. 2B shows a further enlarged part of Fig. 2A.
Figs. 2C and 2D each show a stained tissue image obtained by immunostaining a tissue from the large intestine to which a bioadhesive sheet-shaped material has been attached, with the use of an anti-desmin antibody. Fig. 2D shows a further enlarged part of Fig. 2C.

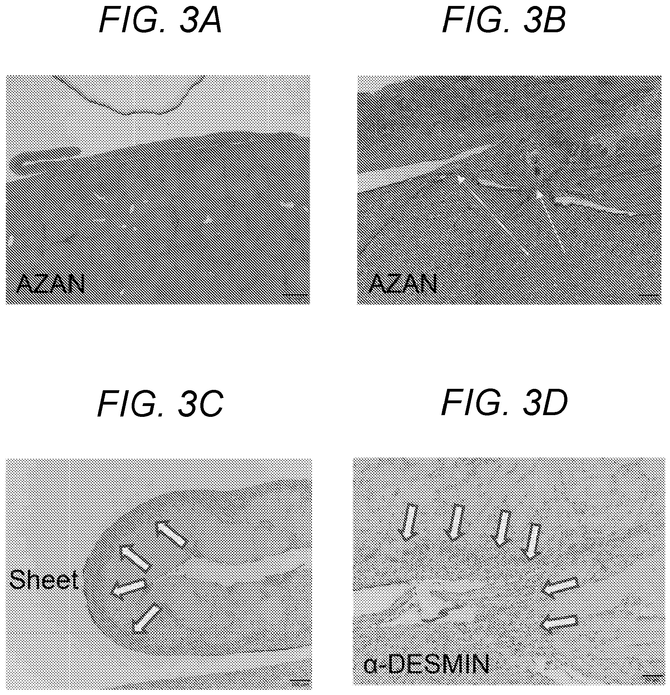

Figs. 3A and 3B each show a stained tissue image obtained by staining a tissue from the liver to which a bioadhesive sheet-shaped material has been attached, with Azan. Fig. 3B shows a further enlarged part of Fig. 3A. The arrows in the diagram show parts where histological continuity is remarkably observed.
Figs. 3C and 3D each show a stained tissue image obtained by immunostaining a tissue from the liver to which a bioadhesive sheet-shaped material has been attached, with the use of an anti-desmin antibody. Figs. 3C and 3D show images in different fields of view within the same slide. The arrows in the diagram show parts where histological continuity is remarkably observed. Further, a bioadhesive sheet-shaped material was attach to the side expressed by "Sheet" in the diagram.

### Description of Embodiments

The present invention relates to a bioadhesive sheet-shaped material, comprising: an extracellular matrix layer; a sheet-shaped cell culture; and a biodegradable gel layer, wherein the bioadhesive sheet-shaped material has the extracellular matrix layer on one surface and the biodegradable gel layer on the other surface with the sheet-shaped cell culture interposed therebetween, for use in a method of attaching the extracellular matrix layer onto a surface of an organ, wherein the organ is selected from the group consisting of stomach, large intestine, duodenum, and liver, wherein the sheet-shaped cell culture contains myoblast cells or mesenchymal stem cells, and wherein the biodegradable gel layer is obtained by mixing a liquid containing fibrinogen with a liquid containing thrombin.

In the present invention, the expression "bioadhesive sheet-shaped material" refers to sheet-shaped one that is used by being attached to a subject with favorable bioadhesiveness. The bioadhesive sheet-shaped material that is for use in a method of the present invention includes an extracellular matrix layer, a sheet-shaped cell culture containing myoblast cells or mesenchymal stem cells, and a biodegradable gel layer wherein this biodegradable layer is obtained by mixing a liquid containing fibrinogen with a liquid containing thrombin, and has the extracellular matrix layer on one surface of the sheet-shaped cell culture contained in the bioadhesive sheet-shaped material and the biodegradable gel layer on the other surface with the sheet-shaped cell culture interposed therebetween.

In the present invention, the expression "favorable bioadhesiveness" means, for example, that a bioadhesive sheet-shaped material and an organ to which the bioadhesive sheet-shaped material has been attached are histologically continuous with each other. The expression "a bioadhesive sheet-shaped material and an organ are histologically continuous with each other" means that a bioadhesive sheet-shaped material and an organ are continuously in contact with each other without leaving any space between them. In this regard, the expression "without leaving any space between them" means that a bioadhesive sheet-shaped material and an organ are at least partially in contact with each other without forming any space between them. For example, in order to eliminate a space between a bioadhesive sheet-shaped material and an organ, the bioadhesive sheet-shaped material and the organ may be sutured. In a case where there is a space between a bioadhesive sheet-shaped material and an organ, other cells infiltrate the space, an effect of the sheet-shaped cell culture in the bioadhesive sheet-shaped material, such as a perforation prevention effect, a liquid leakage prevention effect from the organ, or a therapeutic effect against fibrosis is reduced, and the like can be generated. As to the bioadhesive sheet-shaped material that is for use in a method of the present invention, since an extracellular matrix layer (also referred to as ECM) is attached onto a surface of an organ, there is the extracellular matrix layer between the sheet-shaped cell culture in the bioadhesive sheet-shaped material and the organ. In a part where the bioadhesive sheet-shaped material is in contact with an organ, it can be considered that cells composing the bioadhesive sheet-shaped material migrate into the organ, or on the contrary, cells composing the organ enter the bioadhesive sheet-shaped material. At this time, it can be considered that cells migrate and enter to target the extracellular matrix.

In the present invention, the expression "extracellular matrix layer" refers to one obtained by forming extracellular matrix in a layer form, the extracellular matrix layer contributes to the adhesion of a bioadhesive sheet-shaped material to an organ of a subject, and improves the survival rate of the bioadhesive sheet-shaped material at an attachment site when the bioadhesive sheet-shaped material is attached. Further, the extracellular matrix is a cell-derived component, and thus there is no problem in using the bioadhesive sheet-shaped material of the present invention for attachment to an organ of a subject together with the contained extracellular matrix layer. In the present invention, the thickness of the extracellular matrix layer is 1 nm to 100 nm, and preferably 1 nm to 50 nm.

In the present invention, the expression "sheet-shaped cell culture" means one in a sheet shape formed by connecting cells to each other. The cells may be connected to each other directly (including connection through a cellular element such as an adhesion molecule) and/or through a mediator. The mediator is not particularly limited as long as it is a substance that can at least physically (mechanically) connect cells to each other, and as the mediator, for example, an extracellular matrix or the like can be mentioned. The mediator is preferably derived from a cell, and particularly derived from a cell that forms a cell culture. The cells are at least physically (mechanically) connected to each other, but may be more functionally, for example, chemically electrically connected to each other. The sheet-shaped cell culture may be formed of one cell layer (single layer), or may also be formed of two or more cell layers (such as a laminated (multilayer) body, for example, two-layer, three-layer, four-layer, five-layer, or six-layer). Further, the sheet-shaped cell culture may have a three-dimensional structure having a thickness exceeding the thickness of one cell without showing any clear layered structure of the cells. For example, in the vertical section of the sheet-shaped cell culture, the cells may be present in a state of being non-uniformly (for example, mosaic-like) arranged without being uniformly aligned in the horizontal direction.

The sheet-shaped cell culture preferably does not contain a scaffold (support). The scaffold may be used in some cases in the technical field to which the invention belongs in order to attach cells onto the surface of and/or to the inside of the scaffold and to maintain the physical integrity of the sheet-shaped cell culture, and as the scaffold, for example, a membrane made of polyvinylidene difluoride (PVDF) or the like is known, but the sheet-shaped cell culture contained in the bioadhesive sheet-shaped material that is for use in a method of the present invention can maintain the physical integrity even without such a scaffold. Further, the sheet-shaped cell culture contained in the bioadhesive sheet-shaped material that is for use in a method of the present invention preferably consists only of cell-derived substances making up the sheet-shaped cell culture, and does not contain any other substances.

In the present invention, the thickness of the sheet-shaped cell culture is not particularly limited. In a case where a single-layer sheet is used as the sheet-shaped cell culture, the thickness is usually a thickness of one or more cells, and varies depending on the kind of the cells forming the sheet-shaped cell culture, and in one embodiment, the sheet-shaped cell culture of the present invention has a thickness of 30 µm or more, and in one preferred embodiment, the sheet-shaped cell culture has a thickness of 50 µm or more. Examples of the range of the value of the sheet-shaped cell culture that is contained in the bioadhesive sheet-shaped material for use in a method of the present invention include 30 µm to 200 µm, preferably 50 µm to 150 µm, and more preferably 60 µm to 100 µm. In a case where a laminated sheet is used as the sheet-shaped cell culture, the thickness does not exceed the value obtained by a thickness of the single-layer sheet × the number of laminated sheets. Accordingly, as one embodiment, in a case where, for example, a sheet obtained by stacking five single-layer sheets in layers is used, the sheet has a thickness of 150 µm or more, and in one preferred embodiment, the sheet has a thickness of 250 µm or more. In that case, examples of the range of the value of the sheet-shaped cell culture that is contained in the bioadhesive sheet-shaped material for use in a method of the present invention include 150 µm to 1000 µm, preferably 250 µm to 750 µm, and more preferably 300 µm to 500 µm.

The cells contained in the bioadhesive sheet-shaped material can be derived from any organism used for attachment of the bioadhesive sheet-shaped material, and examples of the organism include, but are not limited to, a human, primates, a dog, a cat, a pig, a horse, a goat, a sheep, a rodent animal (for example, a mouse, a rat, a hamster, or a guinea pig), and a rabbit. Further, as the cells contained in the bioadhesive sheet-shaped material, only one kind of, or two or more kinds of cells may be used. In a preferred embodiment of the present invention, in a case where there are two or more kinds of the cells contained in the bioadhesive sheet-shaped material, the content ratio (purity) of the most abundant cells is 60% or more, preferably 70% or more, and more preferably 75% or more, at the end of the production of the bioadhesive sheet-shaped material.

The cells may be cells derived from heterogeneous cells, or may also be cells derived from homogeneous cells. In this regard, the expression "cells derived from heterogeneous cells" means cells derived from an organism of a species different from that of the recipient to which the bioadhesive sheet-shaped material is attached. For example, in a case where the recipient is a human, cells derived from a monkey or a pig correspond to the cells derived from heterogeneous cells. Further, the expression "cells derived from homogeneous cells" means cells derived from an organism of the same species as that of the recipient. For example, in a case where the recipient is a human, human cells correspond to the cells derived from homogeneous cells. The cells derived from homogeneous cells include self-derived cells (also referred to as "self cells" or "autologous cells"), that is, recipient-derived cells, and cells derived from homogeneous non-self cells (also referred to as "non-autologous cells"). The self-derived cells are preferable in the present invention because the self-derived cells do not cause any rejection even when being transplanted. However, cells derived from heterogeneous cells, or cells derived from homogeneous non-self cells can also be used. In a case where such cells derived from heterogeneous cells or derived from homogeneous non-self cells are used, the cells may require immunosuppressive treatment in order to suppress the rejection, in some cases. Note that in the present specification, the cells other than the self-derived cells, that is, cells derived from heterogeneous cells and cells derived from homogeneous non-self cells may also be collectively referred to as "non-self-derived cells". In one embodiment of the present invention, the cells are autologous cells or non-autologous cells. In one embodiment of the present invention, the cells are autologous cells. In another embodiment of the present invention, the cells are non-autologous cells.

The sheet-shaped cell culture contained in the bioadhesive sheet-shaped material that is for use in a method of the present invention can be produced by any method known to a person skilled in the art (see, for example, Patent Literature **1,** JP 2010-081829 A**,** JP 2011-110368 A**,** and the like). The method for producing a sheet-shaped cell culture typically includes, but is not limited to, a step of seeding cells on a substrate, a step of forming the seeded cells into a sheet-shaped cell culture, and a step of detaching the formed sheet-shaped cell culture from the substrate. Before the step of seeding cells on a substrate, a step of freezing cells and a step of thawing the cells may be performed. Further, a step of washing the cells may be performed after the step of thawing the cells. Each of these steps can be performed by any known technique suitable for the production of a sheet-shaped cell culture. The step of producing a sheet-shaped cell culture may include 1 or 2 or more of the steps according to the above method for producing a sheet-shaped cell culture as the sub-steps. In one embodiment, a step of proliferating the cells is not included after the step of thawing the cells and before the step of seeding the cells on a substrate.

Examples of the substrate are not particularly limited as long as cells can form a cell culture on the substrate, and include containers made of various materials, and a solid or half-solid surface in a container. It is preferable that the container has a structure/material that does not allow a liquid such as a liquid culture medium to permeate. Examples of the material include, but are not limited to, polyethylene, polypropylene, Teflon (registered trademark), polyethylene terephthalate, polymethyl methacrylate, nylon-6,6, polyvinyl alcohol, cellulose, silicon, polystyrene, glass, polyacrylamide, polydimethyl acrylamide, and a metal (for example, iron, stainless steel, aluminum, copper, or brass). Further, it is preferable that the container has at least one flat surface. As such a container, without any limitation, for example, a culture container provided with the bottom made of a substrate capable of forming a cell culture and the liquid-impermeable side can be mentioned. Specific examples of the culture container include, but are not limited to, a cell-culture dish, and a cell-culture bottle. The bottom of the container may be transparent or opaque. If the bottom of a container is transparent, cells can be observed and counted from the underside of the container. Further, the container may have a solid or half-solid surface inside thereof. Examples of the solid surface include a plate, and a container, which are made of various materials as described above, and examples of the half-solid surface include a gel, and a soft polymer matrix. As the substrate, a substrate prepared by using the above-described materials may be used, or a commercially available material may be used. As the preferred substrate, without any limitation, for example, a substrate having an adhesive surface, which is suitable for forming a sheet-shaped cell culture, can be mentioned. Specifically, a substrate having a hydrophilic surface, for example, a substrate of which the surface is coated with a hydrophilic compound such as corona discharge-treated polystyrene, a collagen gel, or a hydrophilic polymer, a substrate of which the surface is coated with an extracellular matrix of collagen, fibronectin, laminin, vitronectin, proteoglycan, glycosaminoglycan, or the like, or a cell adhesion factor such as a cadherin family, a selectin family, or an integrin family, or the like can be mentioned. Further, such a substrate is commercially available (for example, Corning (registered trademark) TC-Treated Culture Dish, Corning, or the like). The overall or part of the substrate may be transparent or opaque.

The surface of the substrate may be coated with a material of which the physical properties change in response to a stimulus, for example, temperature or light. As such a material, without any limitation, a known material, for example, a temperature-responsive material made of a homopolymer or a copolymer of a (meth)acrylamide compound, a N-alkyl-substituted (meth)acrylamide derivative (for example, N-ethyl acrylamide, N-n-propyl acrylamide, N-n-propyl methacrylamide, N-isopropyl acrylamide, N-isopropyl methacrylamide, N-cyclopropyl acrylamide, N-cyclopropyl methacrylamide, N-ethoxyethyl acrylamide, N-ethoxyethyl methacrylamide, N-tetrahydrofurfuryl acrylamide, N-tetrahydrofurfuryl methacrylamide, or the like), a N,N-dialkyl-substituted (meth)acrylamide derivative (for example, N,N-dimethyl (meth)acrylamide, N,N-ethyl methyl acrylamide, N,N-diethyl acrylamide, or the like), a (meth)acrylamide derivative having a cyclic group (for example, 1-(1-oxo-2-propenyl)-pyrrolidine, 1-(1-oxo-2-propenyl)-piperidine, 4-(1-oxo-2-propenyl)-morpholine, 1-(1-oxo-2-methyl-2-propenyl)-pyrrolidine, 1-(1-oxo-2-methyl-2-propenyl)-piperidine, 4-(1-oxo-2-methyl-2-propenyl)-morpholine, or the like), or a vinyl ether derivative (for example, methyl vinyl ether), or a photoresponsive material such as a light-absorbing polymer having an azobenzene group, a copolymer of a vinyl derivative of triphenylmethane leucohydroxide and an acrylamide-based monomer, or N-isopropylacrylamide gel containing spirobenzopyran, may be used (see, for example, JP H02-211865 A, and JP 2003-33177 A). By giving a predetermined stimulus to such a material, the physical properties, for example, the hydrophilicity and the hydrophobicity can be changed, and the detachment of a cell culture attached on the material can be promoted. A culture dish coated with a temperature-responsive material is commercially available (for example, UpCell (registered trademark) of CellSeed Inc., or Cepallet (registered trademark) of DIC Corporation), and such a culture dish can be used in the production method of the present disclosure.

The substrate may have various shapes, but is preferably flat. Further, the area is not particularly limited, and may be, for example, around 1 cm² to around 200 cm², around 2 cm² to around 100 cm², or around 3 cm² to around 50 cm². For example, as the substrate, a circular culture dish having a diameter of 10 cm can be mentioned. In this case, the area is 56.7 cm².

The substrate may be coated with serum. By using a substrate coated with serum, a sheet-shaped cell culture with a higher density can be formed. The expression "coated with serum" means a state that a serum component adheres onto a surface of the substrate. Such a state can be obtained, for example, by processing a substrate with serum, without any limitation. The processing with serum includes bringing serum into contact with a substrate, and performing the incubation for a predetermined period of time as needed.

As the serum, heterologous serum and/or homologous serum can be used. In a case where a bioadhesive sheet-shaped material is used by the attachment, the heterologous serum means serum derived from an organism of a species different from that of the recipient. For example, in a case where the recipient is a human, serum derived from a bovine or a horse, for example, fetal bovine serum/fetal calf serum (FBS/FCS), calf serum (CS), horse serum (HS), or the like corresponds to the heterologous serum. Further, the expression "homologous serum" means serum derived from an organism of the same species as that of the recipient. For example, in a case where the recipient is a human, human serum corresponds to the homologous serum. The homologous serum includes self serum (also referred to as "autologous serum"), that is, serum derived from the recipient, and homologous non-autologous serum derived from an individual of the same species other than the recipient. Note that in the present specification, serum other than self serum, that is, heterologous serum and homologous non-autologous serum may be collectively referred to as "non-self serum".

The serum for coating on a substrate is commercially available, or can be prepared from the blood collected from a desired organism by a conventional method. Specifically, for example, a method in which the collected blood is left to stand at room temperature for around 20 minutes to around 60 minutes so as to be coagulated, the coagulated blood is centrifuged at around 1000×g to around 1200×g, and a supernatant is collected, or the like can be mentioned.

In a case where the incubation is performed on a substrate, serum may be used in undiluted form, or may be diluted for use. The serum can be diluted, without any limitation, in any medium, for example, water, a saline solution, various buffer solutions (for example, PBS, HBSS and the like), various liquid media (for example, DMEM, MEM, F12, DMEM/F12, DME, RPMI1640, MCDB (such as MCDB102, 104, 107, 120, 131, 153, or 199), L15, SkBM, or RITC80-7) or the like. The dilution concentration is not particularly limited as long as the serum component can adhere onto a substrate, and is, for example, around 0.5% to around 100% (v/v), preferably around 1% to around 60% (v/v), and more preferably around 5% to around 40% (v/v).

The incubation time is also not particularly limited as long as the serum component can adhere onto a substrate, and is, for example, around 1 hour to around 72 hours, preferably around 2 hours to around 48 hours, more preferably around 2 hours to around for 24 hours, and furthermore preferably around 2 hours to around 12 hours. The incubation temperature is also not particularly limited as long as the serum component can adhere onto a substrate, and is, for example, around 0°C to around 60°C, preferably around 4°C to around 45°C, and more preferably room temperature to around 40°C.

The serum may be discarded after incubation. As the technique for discarding the serum, suction with a pipette or the like, or a conventionally-used technique for discarding liquid such as decantation can be used. In a preferred embodiment of the present disclosure, after the serum is discarded, the substrate may be washed with a serum-free washing solution. The serum-free washing solution is not particularly limited as long as it is a liquid medium that does not contain serum and does not adversely affect the serum component adhered onto a substrate, and the washing can be performed, without any limitation, for example, by water, a saline solution, various buffer solutions (for example, PBS, HBSS and the like), various liquid media (for example, DMEM, MEM, F12, DMEM/F12, DME, RPMI1640, MCDB (such as MCDB102, 104, 107, 120, 131, 153, or 199), L15, SkBM, or RITC80-7), or the like. As the washing technique, without any limitation, a conventionally-used technique for washing a substrate, for example, a technique in which a serum-free washing solution is added onto a substrate, stirred for a predetermined time (for example, around 5 seconds to around 60 seconds), and then discarded, or the like can be used.

In the present invention, the substrate may be coated with a growth factor. In this regard, the expression "growth factor" means any substance that promotes proliferation of cells as compared with a substrate that is not coated with the growth factor, and examples of the growth factor include epidermal growth factor (EGF), vascular endothelial growth factor (VEGF), and fibroblast growth factor (FGF). The technique for coating on a substrate with a growth factor, the discarding technique, and the washing technique are basically the same as those of serum except that the dilution concentration at the time of incubation is, for example, around 0.000 1 µg/mL to around 1 µg/mL, preferably around 0.0005 µg/mL to around 0.05 µg/mL, and more preferably around 0.001 µg/mL to around 0.01 µg/mL.

In the present invention, the substrate may be coated with a steroid drug. In this regard, the expression "steroid drug" refers to a compound that could exert an adverse effect on the living body, such as adrenocortical insufficiency, or Cushing syndrome, among the compounds having a steroid nucleus. Examples of the compound include, but are not limited to, cortisol, prednisolone, triamcinolone, dexamethasone, and betamethasone. The technique for coating on a substrate with a steroid drug, the discarding technique, and the washing technique are basically the same as those of serum except that the dilution concentration at the time of incubation is, for example, 0.1 µg/mL to around 100 µg/mL, preferably around 0.4 µg/mL to around 40 µg/mL, and more preferably around 1 µg/mL to around 10 µg/mL, as dexamethasone.

The substrate may be coated with any one of the serum, the growth factor, and the steroid drug, or may be coated with any combination of the serum, the growth factor, and the steroid drug, that is, a combination of the serum and the growth factor, a combination of the serum and the steroid drug, a combination of the serum, the growth factor, and the steroid drug, or a combination of the growth factor and the steroid drug. In a case of coating with multiple components, the coating with these components may be performed at the same time by mixing the components with each other, or may be performed in separate steps.

The substrate may be seeded with cells immediately after being coated with serum and the like, or may also be stored after being coated and then seeded with cells. The coated substrate can be stored for a long period of time, for example, by keeping the substrate at around 4°C or less, preferably around -20°C or less, and more preferably around -80°C or less.

Seeding of cells on a substrate can be performed by any known technique under any known conditions. The seeding of cells on a substrate may be performed, for example, by injecting a cell suspension in which cells are suspended in a liquid culture medium into a substrate (culture container). For the injection of the cell suspension, an instrument suitable for the injection operation of the cell suspension, such as a dropper or a pipette, can be used.

In one embodiment, the seeding can be performed at a density of around 7.1 × 10⁵ cells/cm² to around 3.0 × 10⁶ cells/cm², around 7.3 × 10⁵ cells/cm² to around 2.8 × 10⁶ cells/cm², around 7.5 × 10⁵ cells/cm² to around 2.5 × 10⁶ cells/cm², around 7.8 × 10⁵ cells/cm² to around 2.3 × 10⁶ cells/cm², around 8.0 × 10⁵ cells/cm² to around 2.0 × 10⁶ cells/cm², around 8.5 × 10⁵ cells/cm² to around 1.8 × 10⁶ cells/cm², around 9.0 × 10⁵ cells/cm² to around 1.6 × 10⁶ cells/cm², around 1.0 × 10⁶ cells/cm² to around 1.6 × 10⁶ cells/cm², or the like.

In the present invention, the expression "biodegradable gel layer" refers to one in a layer form of biodegradable gel that is a gel being degraded in the body, absorbed into the body, metabolized, and excreted. Examples of the biodegradable gel include, but are not limited to, a fibrin gel, and a gel formed by Adspray (registered trademark) (manufactured by TERUMO CORPORATION). The biodegradable gel refers to a gel that generates viscosity by mixing preferably two kinds of liquids. For example, the fibrin gel is a gel having high strength formed by the action of thrombin on fibrinogen by mixing a liquid containing the fibrinogen (hereinafter, referred to as "fibrinogen solution") with a liquid containing the thrombin (hereinafter, referred to as "thrombin solution").

Further, since the biodegradable gel can be degraded in the body, the bioadhesive sheet-shaped material of the present invention can be used for the attachment to an organ of a subject together with the contained biodegradable gel layer. In the present invention, the thickness of the biodegradable gel layer is 5 µm to 1300 µm, and preferably 50 µm to 300 µm.

In one embodiment, the thickness of the biodegradable gel layer is uniform. In this regard, the term "uniform" refers that the difference between the thickest part and the thinnest part is 20% or less. The term "uniform" refers that the difference in the thickness is preferably 10% or less, and more preferably 5% or less.

The bioadhesive sheet-shaped material that is for use in a method of the present invention is used by attaching an extracellular matrix layer contained in the bioadhesive sheet-shaped material onto a surface of an organ. The organ to which the bioadhesive sheet-shaped material that is for use in a method of the present invention is attached is selected from the group consisting of stomach, large intestine, duodenum, and liver. In the present invention, the term "organ" refers to an organ having specific morphology and function, which is within the body cavity, and examples of the organ include a digestive organ such as liver, gallbladder, large intestine, stomach, or duodenum, a circulatory organ such as heart, or spleen, a respiratory organ such as lung, a urinary organ such as kidney, a genital organ such as prostate, uterus, or ovary, and an endocrine organ such as adrenal gland. It is considered that oxygen, nutrients, and the like need to be supplied to a sheet-shaped cell culture in order to engraft an organ with the sheet-shaped cell culture and to retain the organ in a favorable condition, and since the bioadhesive sheet-shaped material of the present invention contains an extracellular matrix layer, it is considered that the bioadhesive sheet-shaped material releases more various cytokines such as VEGF, and HGF, which have angiogenic activity.

In one embodiment, the attachment of the bioadhesive sheet-shaped material for use in a method of the present invention onto a surface of an organ is attachment under a laparoscope or a thoracoscope. In this regard, the expression "attachment under a laparoscope or a thoracoscope" means that the bioadhesive sheet-shaped material is delivered to the vicinity of an organ of a subject through a path of an instrument (for example, a port for a laparoscope, a port for a thoracoscope, or the like) prepared for laparoscopic or thoracoscopic surgery in a subject, and attached onto a surface of the organ. When the attachment is performed under a laparoscope or a thoracoscope, the bioadhesive sheet-shaped material may be curved with forceps or the like, and the curved bioadhesive sheet-shaped material may be passed through a path of an instrument, and spread in the vicinity of an organ so as to be attached to the organ. Further, when the bioadhesive sheet-shaped material is passed through a path of an instrument in this way, a device for delivery may be used. As the device for delivery, for example, a therapeutic-substance carrying/administering appliance (see, JP 2009-000511 A), or the like can be used, although the device is not limited to such an appliance.

Accordingly, in one embodiment, the organ to which the bioadhesive sheet-shaped material for use in a method of the present invention is attached is an organ that can be subjected to laparoscopic surgery or thoracoscopic surgery, that is, an organ in the body, capable of being approached from outside the body by a laparoscope or a thoracoscope, and the organ is selected from the group consisting ofstomach, large intestine, duodenum, and liver.

The cells contained in the bioadhesive sheet-shaped material that is for use in a method of the present invention form a sheet-shaped cell culture, and can produce an extracellular matrix. The cells contained in the bioadhesive sheet-shaped material for use in a method of the present invention are mesenchymal stem cells or myoblast cells, and more preferably myoblast cells.

The myoblast cells are well known in the technical field to which the present invention belongs, and can be prepared from skeletal muscle by any known method (for example, method disclosed in JP 2007-89442 A, or the like), and as the myoblast cells, for example, catalog number: CC-2580 of Lonza Japan, product code 3520 of Cosmo Bio Co., Ltd., or the like can be obtained commercially. The myoblast cells are not limited to such cells, and can be identified by a marker such as CD56, α7 integrin, myosin heavy chain IIa, myosin heavy chain IIb, myosin heavy chain IId (IIx), MyoD, Myf5, Myf6, myogenin, desmin, or PAX3. In one embodiment, the myoblast cells are CD56 positive. In one embodiment, the myoblast cells are CD56 positive and desmin positive.

In a case where the myoblast cells are prepared from striated muscle tissue, the prepared cell population contains fibroblast cells. When the cell culture for use in a method according to the present invention is produced, in a case where a cell population containing the myoblast cells prepared from striated muscle tissue is used, a certain amount of fibroblast cells is contained in the cell population. The fibroblast cells are well known in the technical field to which the present invention belongs, and can be identified by a marker such as TE-7 (see, for example, Rosendaal et al., J Cell Sci. 1994, 107(Pt1): 29-37, Goodpaster et al. J Histochem Cytochem. 2008, 56(4): 347-358, and the like).

In one embodiment, the cells forming the cell culture for use in a method of the present invention include the myoblast cells prepared from striated muscle tissue. Accordingly, the cell population used in production of the cell culture for use in a method of the present invention can contain myoblast cells and fibroblast cells. In one embodiment, the cell population used in production of the cell culture for use in a method of the present invention can have a CD56-positive rate of 50% or more, 60% or more, 65% or more, 70% or more, 75% or more, 80% or more, 85% or more, or 90% or more, and preferably 60% or more.

In a case where the bioadhesive sheet-shaped material for use in a method of the present invention contains myoblast cells, the cell population used in production of the bioadhesive sheet-shaped material can include fibroblast cells, but in a case where the content of the fibroblast cells is extremely high, the content of myoblast cells is lowered, and thus, this is not preferable. Accordingly, in one embodiment, the cell population used in production of the cell culture for use in a method of the present invention can have a TE-7 positive rate of 50% or less, 40% or less, 35% or less, 30% or less, 25% or less, 20% or less, 15% or less, or 10% or less, and preferably 40% or less.

The cell population used in production of the bioadhesive sheet-shaped material for use in a method of the present invention can contain cells other than the myoblast cells and the fibroblast cells, and the smaller the number of such cells is, the more preferable the cell population is. Accordingly, the higher the total value of the CD56-positive rate and the TE-7 positive rate is, the more preferable the cell population is, and the total value can be, for example, 80% or more, 85% or more, 90% or more, 95% or more, 96% or more, 97% or more, 98% or more, or 99% or more, and preferably 90% or more.

In one embodiment, the bioadhesive sheet-shaped material for use in a method of the present invention further contains a medical component. In this regard, the expression "medical component" means any component that treats abnormalities of an organ engrafted with the bioadhesive sheet-shaped material. Examples of the abnormalities of an organ include, but are not limited to, inflammation generated in an organ, tumor formed in an organ, and ulcer formed in an organ, and examples of the medical component to treat the abnormalities of such an organ include, but are not limited to, an anti-inflammatory agent, an antimicrobe agent, an antifungal agent, an antihistamine, an adrenal cortical hormone, an anticancer agent, and any combination thereof.

In addition to an extracellular matrix layer, a sheet-shaped cell culture, a biodegradable gel layer, and/or a medical component, the bioadhesive sheet-shaped material for use in a method of the present invention may contain various additional components, for example, a pharmaceutically acceptable carrier, a component that enhances the survivability, bioadhesiveness, function, and/or the like of the bioadhesive sheet-shaped material, a component that assists the medical component useful for treatment of abnormalities of an organ of a subject, and the like. As such additional components, any known additional components can be used, and a person skilled in the art is well-versed in the additional components. Further, the bioadhesive sheet-shaped material for use in a method of the present invention can be used in combination with the component that enhances the survivability, bioadhesiveness, function, and/or the like of the bioadhesive sheet-shaped material, the component that assists the medical component useful for treatment of abnormalities of an organ of a subject, and the like.

Further disclosed herein is a method for producing a bioadhesive sheet-shaped material, including: a step of seeding cells on a substrate; a step of forming the seeded cells into a sheet shape; and a step of detaching the formed sheet-shaped cell culture from the substrate.

The sheet-shaped cell culture contained in the bioadhesive sheet-shaped material that is used in a method of the present invention can be produced by any method known to a person skilled in the art (see, for example, Patent Literature 1, JP 2010-081829 A, JP 2011-110368 A, and the like) as described above. The method for producing a sheet-shaped cell culture typically includes, but is not limited to, a step of seeding cells on a substrate, a step of forming the seeded cells into a sheet-shaped cell culture, and a step of detaching the formed sheet-shaped cell culture from the substrate.

The method for producing a bioadhesive sheet-shaped material that is for use in a method of the present invention may further include a step of forming an extracellular matrix layer. In the present invention, although depending on various conditions, an extracellular matrix forming an extracellular matrix layer is produced, for example, by seeding cells, and then culturing the seeded cells for 24 hours or more, for example, for 24 hours, 30 hours, 36 hours, 42 hours, 48 hours, 54 hours, 60 hours, 66 hours, or 72 hours, which are contained in a sheet-shaped cell culture. The produced extracellular matrix is formed in a layer form, for example, between the sheet-shaped cell culture and a substrate. In this case, in the subsequent step of detaching the formed sheet-shaped cell culture from the substrate, the sheet-shaped cell culture is preferably detached from the substrate without damaging the formed extracellular matrix layer. The method for detaching the sheet-shaped cell culture from the substrate so as not to damage the formed extracellular matrix layer includes, but is not limited to, using a culture dish coated with a temperature-responsive material for the culture.

The method for producing a bioadhesive sheet-shaped material that is for use in a method of the present invention may further include a step of forming a biodegradable gel layer. As the method for forming a biodegradable gel layer includes, for example, although the method is not limited to, a method for forming a fibrin gel layer by adding a fibrinogen solution dropwise onto a sheet-shaped cell culture, and then spraying a thrombin solution (see, JP 6495603 B2), or the like can be mentioned. The method for producing a bioadhesive sheet-shaped material that is for use in a method of the present invention may further include a step of adding a medical component. The step of adding a medical component may be performed before or after the step of forming the seeded cells into a sheet shape, or may be performed after the step of forming the biodegradable gel layer.

Further disclosed herein is a method for treating a disease that is ameliorated by application of the bioadhesive sheet-shaped material, including attaching a surface having an extracellular matrix layer of the bioadhesive sheet-shaped material in an effective amount to an organ of a subject in need thereof.

In the present invention, the term "subject" means any individual organism, preferably an animal, more preferably a mammal, and furthermore preferably an individual human.

Further, the term "treatment" includes cure of disease, and all types of medically acceptable prophylactic and/or therapeutic interventions for the purpose of temporary remission, prophylaxis or the like. For example, the term "treatment" includes medically acceptable intervention for the various purposes, including retardation or suspension of the progression of disease, regression or disappearance of lesion, prevention of the onset or recurrence of the disease, and the like.

In the present invention, the term "effective amount" means, for example, an amount (for example, the number of the cells contained in a bioadhesive sheet-shaped material, the size, weight, or the like of the sheet-shaped cell culture contained in a bioadhesive sheet-shaped material) with which the onset or recurrence of disease can be suppressed, the symptoms can be alleviated, or the progression can be retarded or suspended, and preferably an amount with which the onset or recurrence of disease is prevented, or the disease is cured. Further, an amount with which any adverse effect exceeding the benefit of administration is not exerted is preferable. Such an amount can be appropriately determined by, for example, a test or the like in an experimental animal or a disease model animal such as a mouse, a rat, a dog, or a pig, and such a test method is well known to a person skilled in the art. In addition, the size of the histological lesion to be treated can be an important indicator for determining the effective amount.

In accordance with the production method as disclosed herein, the treatment method as disclosed herein may further include a step of producing a bioadhesive sheet-shaped material for use in a method of the present invention. The treatment method may further include a step of collecting cells (for example, skin cells, blood cells, or the like in a case of using iPS cells) for producing a sheet-shaped cell culture contained in a bioadhesive sheet-shaped material, or tissue (for example, skin tissue, blood, or the like in a case of using iPS cells) that is a supply source of cells, from a subject before the step of producing the bioadhesive sheet-shaped material. In one embodiment, a subject from which the cells or the tissue to be a supply source of cells is collected is the same individual as a subject to which a bioadhesive sheet-shaped material is attached. In another embodiment, a subject from which the cells or the tissue to be a supply source of cells is collected is another individual of the same species as that of a subject to which a bioadhesive sheet-shaped material is attached. Further, in another embodiment, a subject from which the cells or the tissue to be a supply source of cells is collected is an individual of a species different from that of a subject to which a bioadhesive sheet-shaped material is attached.

As the attachment method, typically, direct attachment to tissue can be mentioned, and for example, direct attachment to an organ of a subject to be attached. The method for attaching the bioadhesive sheet-shaped material that is for use in a method of the present invention may be, for example, attachment under a laparoscope or a thoracoscope, in addition to the above attachment. In this regard, the expression "attachment under a laparoscope or a thoracoscope" means that the bioadhesive sheet-shaped material is delivered to the vicinity of an organ of a subject through a path of an instrument (for example, a port for a laparoscope, a port for a thoracoscope, or the like) prepared for laparoscopic or thoracoscopic surgery, and attached onto a surface of an organ of a subject, and such delivery to the vicinity of an organ of a subject may be performed by using a device for delivery. The device for delivery may be, for example, a device including a support member for supporting a bioadhesive sheet-shaped material, and a protective member with a low coefficient of friction for protecting one surface of the support, in which the support supporting the bioadhesive sheet-shaped material is protected with the protective member, and the protected support is rolled up so as to be inserted into a cylindrical body.

### Examples

The present invention will be described in more detail with reference to the following Examples, which shows specific examples of the present invention, and the present invention is not limited thereto.

### Example 1: Preparation of sheet-shaped cell culture

The striated muscle in the lower limb of a pig is collected under general anesthesia, and the collected tissue is processed with an enzymatic digestive juice containing collagenase and trypsin, and dispersed in single cells. Such cells were cultured in a 20% FBS-containing MCDB131 medium under the conditions of 37°C and 5% CO₂ until the confluency was obtained, and the cells were collected. 2.2 × 10⁷ collected cells were seeded in a 60-mm temperature-responsive culture dish (UpCell (registered trademark), 6-cm dish, CS3006, CellSeed Inc.), cultured in a 20% FBS-containing DMEM/F12 medium for 12 hours, and the sheet-forming and the forming of an extracellular matrix layer were performed. After that, by lowering the temperature to 20°C, a sheet-shaped cell culture was detached and collected from the culture dish while having the extracellular matrix layer. Onto a surface on the side opposite to the surface having the extracellular matrix layer of the sheet-shaped cell culture, 500 µL of fibrinogen solution (in which a contained material (lyophilized fibrinogen powder) of Vial 1 of Bolheal (registered trademark) tissue adhesion bond (Teijin Pharma Limited.) was dissolved with a contained solution (fibrinogen dissolving solution) of Vial 2 at a fibrinogen concentration of 80 mg/mL) was added dropwise, and then 800 µL of thrombin solution (in which a contained material (lyophilized thrombin powder) of Vial 3 of Bolheal (registered trademark) tissue adhesion bond (Teijin Pharma Limited.) was dissolved with a contained solution (thrombin dissolving solution) of Vial 4 at a thrombin concentration of 250 unit/mL) was sprayed. By the reaction in the dropwise addition of the fibrinogen solution and the subsequent spraying of the thrombin solution, a biodegradable gel layer made of fibrin gel was formed on a surface on the side opposite to the surface having an extracellular matrix layer of the sheet-shaped cell culture, and a bioadhesive sheet-shaped material was obtained.

### Example 2: Attachment of bioadhesive sheet-shaped material to organ and collection of tissue

A bioadhesive sheet-shaped material prepared in Example 1 was attached under general anesthesia to a pig from which the striated muscle had been collected in Example 1. The attachment was performed so as to attach a surface of an extracellular matrix layer of the bioadhesive sheet-shaped material onto a surface of the stomach, the large intestine, or the liver. The pig to which the bioadhesive sheet-shaped material had been attached was subjected to laparotomy again under general anesthesia on day 3 after the attachment, the attachment site was observed, and then the tissue was collected.

### Example 3: Observation of stained tissue image of collected tissue

Each stained tissue image was obtained from the collected tissue. Stained tissue images that were obtained by staining the tissue from the stomach with hematoxylin-eosin staining are shown in Figs. 1A and 1B. Stained tissue images that were obtained by staining the tissue from the large intestine with hematoxylin-eosin staining are shown in Figs. 2A and 2B, and stained tissue images that were obtained by immunostaining the tissue from the large intestine with the use of an anti-desmin antibody are shown in Figs. 2C and 2D. Stained tissue images that were obtained by staining the tissue from the liver with Azan are shown in Figs. 3A and 3B, and stained tissue images that were obtained by immunostaining the tissue from the liver with the use of an anti-desmin antibody are shown in Figs. 3C and 3D.

It has been confirmed from the stained tissue images of Figs. 1 to 3 that the bioadhesive sheet-shaped material is engrafted into an organ of a subject to be attached, and the organ and the bioadhesive sheet-shaped material are histologically continuous with each other, in the stomach, the large intestine, and the liver. In particular, in the part surrounded by a circle with a straight line in Fig. 1B, and the parts indicated by arrows in Figs. 3B, 3C and 3D, it has been remarkably confirmed that the organ and the bioadhesive sheet-shaped material are histologically continuous with each other.

## Claims

1. A bioadhesive sheet-shaped material, comprising: an extracellular matrix layer; a sheet-shaped cell culture; and a biodegradable gel layer, wherein the bioadhesive sheet-shaped material has the extracellular matrix layer on one surface and the biodegradable gel layer on the other surface with the sheet-shaped cell culture interposed therebetween, for use in a method of attaching the extracellular matrix layer onto a surface of an organ, wherein the organ is selected from the group consisting of stomach, large intestine, duodenum, and liver, wherein
the sheet-shaped cell culture contains myoblast cells or mesenchymal stem cells, and wherein
the biodegradable gel layer is obtained by mixing a liquid containing fibrinogen with a liquid containing thrombin.

2. The bioadhesive sheet-shaped material for use according to claim 1, wherein the bioadhesive sheet-shaped material and the organ to which the bioadhesive sheet-shaped material has been attached are histologically continuous with each other.

3. The bioadhesive sheet-shaped material for use according to claim 1 or 2, wherein the organ is a human organ.

4. The bioadhesive sheet-shaped material for use according to any one of claims 1 to 3, wherein the attachment onto a surface of an organ is performed under a laparoscope or a thoracoscope.

5. The bioadhesive sheet-shaped material for use according to any one of claims 1 to 4, wherein the organ is an organ in the body, capable of being approached from outside the body by a laparoscope or a thoracoscope.

6. The bioadhesive sheet-shaped material for use according to any one of claims 1 to 5, further comprising a medical component.

7. A bioadhesive sheet-shaped material as defined in any one of claims 1 to 6, for use in a method for treating a disease that is ameliorated by application of the bioadhesive sheet-shaped material, comprising attaching a surface having an extracellular matrix layer of the bioadhesive sheet-shaped material in an effective amount of the bioadhesive sheet-shaped material to an organ of a subject in need thereof, wherein the organ is selected from the group consisting of stomach, large intestine, duodenum, and liver.

8. The bioadhesive sheet-shaped material for use according to claim 7, wherein the bioadhesive sheet-shaped material and the organ to which the bioadhesive sheet-shaped material has been attached are histologically continuous with each other.

9. The bioadhesive sheet-shaped material for use according to claim 7 or 8, wherein the attachment to an organ of a subject is performed under a laparoscope or under a thoracoscope.

10. The bioadhesive sheet-shaped material for use according any one of claims 7 to 9, wherein
the attachment to an organ of a subject is performed by using a device including:
a support member for supporting a bioadhesive sheet-shaped material; and
a protective member with a low coefficient of friction for protecting one surface of the support, wherein
the support supporting the bioadhesive sheet-shaped material is protected with the protective member, the protected support is rolled up, and the rolled-up support is inserted into a cylindrical body.

## Patentansprüche

1. Bioadhäsives, blattförmiges Material, umfassend: eine extrazelluläre Matrixschicht; eine blattförmige Zellkultur; und eine biologisch abbaubare Gelschicht, wobei das bioadhäsive, blattförmige Material die extrazelluläre Matrixschicht auf einer Oberfläche und die biologisch abbaubare Gelschicht auf der anderen Oberfläche aufweist, mit der blattförmige Zellkultur dazwischen angeordnet, zur Verwendung in einem Verfahren zum Anbringen der extrazellulären Matrixschicht auf einer Oberfläche eines Organs, wobei das Organ aus der Gruppe ausgewählt ist, die aus Magen, Dickdarm, Zwölffingerdarm und Leber besteht, wobei die blattförmige Zellkultur Myoblastenzellen oder mesenchymale Stammzellen enthält und wobei
die biologisch abbaubare Gelschicht durch Mischen einer Flüssigkeit, die Fibrinogen enthält, mit einer Flüssigkeit, die Thrombin enthält, erhalten wird.

2. Bioadhäsives blattförmiges Material zur Verwendung gemäß Anspruch 1, wobei das bioadhäsive blattförmige Material und das Organ, an dem das bioadhäsive blattförmige Material angebracht wurde, histologisch miteinander zusammenhängend sind.

3. Bioadhäsives blattförmiges Material zur Verwendung gemäß Anspruch 1 oder 2, wobei das Organ ein menschliches Organ ist.

4. Bioadhäsives blattförmiges Material zur Verwendung gemäß irgendeinem der Ansprüche 1 bis 3, wobei das Anbringen an einer Oberfläche eines Organs unter einem Laparoskop oder einem Thorakoskop durchgeführt wird.

5. Bioadhäsives blattförmiges Material zur Verwendung gemäß irgendeinem der Ansprüche 1 bis 4, wobei das Organ ein Organ im Körper ist, das von außerhalb des Körpers mit einem Laparoskop oder einem Thorakoskop erreicht werden kann.

6. Bioadhäsives blattförmiges Material zur Verwendung gemäß irgendeinem der Ansprüche 1 bis 5, ferner umfassend eine medizinische Komponente.

7. Bioadhäsives blattförmiges Material wie in irgendeinem der Ansprüche 1 bis 6 definiert, zur Verwendung in einem Verfahren zur Behandlung einer Krankheit, die durch die Anwendung des bioadhäsiven blattförmigen Materials gelindert wird, umfassend das Anbringen einer Oberfläche mit einer extrazellulären Matrixschicht des bioadhäsiven blattförmigen Materials in einer wirksamen Menge des bioadhäsiven blattförmigen Materials an einem Organ eines Subjekts, das dessen bedarf, wobei das Organ aus der Gruppe ausgewählt ist, die aus Magen, Dickdarm, Zwölffingerdarm und Leber besteht.

8. Bioadhäsives blattförmiges Material zur Verwendung gemäß Anspruch 7, wobei das bioadhäsive blattförmige Material und das Organ, an dem das bioadhäsive blattförmige Material angebracht wurde, histologisch miteinander zusammenhängend sind.

9. Bioadhäsives blattförmiges Material zur Verwendung gemäß Anspruch 7 oder 8, wobei das Anbringen an einem Organ eines Subjekts unter einem Laparoskop oder unter einem Thorakoskop durchgeführt wird.

10. Bioadhäsives blattförmiges Material zur Verwendung gemäß irgendeinem der Ansprüche 7 bis 9, wobei
das Anbringen an einem Organ eines Subjekts unter Verwendung einer Vorrichtung durchgeführt wird, die Folgendes umfasst:
ein Stützelement zum Stützen eines bioadhäsiven blattförmigen Materials; und
ein Schutzelement mit einem niedrigen Reibungskoeffizienten zum Schützen einer Oberfläche der Stütze, wobei
die das bioadhäsive blattförmige Material stützende Stütze mit dem Schutzelement geschützt wird, die geschützte Stütze aufgerollt wird und die aufgerollte Stütze in einen zylindrischen Körper eingeführt wird.

## Revendications

1. Matériau bioadhésif en forme de feuille, comprenant : une couche de matrice extracellulaire, une culture cellulaire en forme de feuille, et une couche de gel biodégradable, où le matériau bioadhésif en forme de feuille a la couche de matrice extracellulaire sur une surface et la couche de gel biodégradable sur l'autre surface avec la culture cellulaire en forme de feuille interposée entre elles, pour une utilisation dans un procédé de fixation de la couche de matrice extracellulaire sur une surface d'un organe, où l'organe est choisi dans le groupe constitué par l'estomac, le gros intestin, le duodénum, et le foie, où
la culture cellulaire en forme de feuille contient des cellules de myoblaste ou des cellules souches mésenchymateuses, et où
la couche de gel biodégradable est obtenue en mélangeant un liquide contenant du fibrinogène avec un liquide contenant de la thrombine.

2. Matériau bioadhésif en forme de feuille pour une utilisation selon la revendication 1, où le matériau bioadhésif en forme de feuille et l'organe auquel le matériau bioadhésif en forme de feuille a été fixé sont histologiquement continus l'un avec l'autre.

3. Matériau bioadhésif en forme de feuille pour une utilisation selon la revendication 1 ou 2, où l'organe est un organe humain.

4. Matériau bioadhésif en forme de feuille pour une utilisation selon l'une quelconque des revendications 1 à 3, où la fixation sur une surface d'un organe est effectuée sous un laparoscope ou un thoracoscope.

5. Matériau bioadhésif en forme de feuille pour une utilisation selon l'une quelconque des revendications 1 à 4, où l'organe est un organe dans le corps, capable d'être approché de l'extérieur du corps par un laparoscope ou un thoracoscope.

6. Matériau bioadhésif en forme de feuille pour une utilisation selon l'une quelconque des revendications 1 à 5, comprenant en outre un composant médical.

7. Matériau bioadhésif en forme de feuille tel que défini dans l'une quelconque des revendications 1 à 6, pour une utilisation dans un procédé de traitement d'une maladie qui est améliorée par l'application du matériau bioadhésif en forme de feuille, comprenant la fixation d'une surface ayant une couche de matrice extracellulaire du matériau bioadhésif en forme de feuille dans une quantité efficace du matériau bioadhésif en forme de feuille à un organe d'un sujet en ayant besoin, où l'organe est choisi dans le groupe constitué par l'estomac, le gros intestin, le duodénum, et le foie.

8. Matériau bioadhésif en forme de feuille pour une utilisation selon la revendication 7, où le matériau bioadhésif en forme de feuille et l'organe auquel le matériau bioadhésif en forme de feuille a été fixé sont histologiquement continus l'un avec l'autre.

9. Matériau bioadhésif en forme de feuille pour une utilisation selon la revendication 7 ou 8, où la fixation à un organe d'un sujet est effectuée sous un laparoscope ou sous un thoracoscope.

10. Matériau bioadhésif en forme de feuille pour une utilisation selon l'une quelconque des revendications 7 à 9, où
la fixation à un organe d'un sujet est effectuée en utilisant un dispositif comprenant :
un élément de support pour supporter un matériau bioadhésif en forme de feuille, et
un élément de protection avec un faible coefficient de frottement pour protéger une surface du support, où
le support supportant le matériau bioadhésif en forme de feuille est protégé avec l'élément de protection, le support protégé est enroulé, et le support enroulé est inséré dans un corps cylindrique.
